# EUROPÄISCHE PATENTSCHRIFT

(11) **EP 0 691 334 B1**
(45) Veröffentlichungstag und Bekanntmachung des Hinweises auf die Patenterteilung: **03.12.1997**
(21) Anmeldenummer: 95110199.7
(22) Anmeldetag: 30.06.1995
(51) Int. Cl.: C07D 307/28, B01J 31/02

(54) **Verfahren zur Herstellung von 2,5-Dihydrofuran**
Process for the preparation of 2,5-dihydrofuran
Procédé de préparation de 2,5-dihydrofuranne

(30) Priorität: 09.07.1994 DE 4424219
(43) Veröffentlichungstag der Anmeldung: 10.01.1996
(73) Patentinhaber: BASF Aktiengesellschaft, 67063 Ludwigshafen (DE)
(72) Erfinder: Fischer, Martin, Dr., D-67071 Ludwigshafen (DE)

(56) Entgegenhaltungen:
- EP-A- 0 412 366
- WO-A-93/10111
- CHEMICAL ABSTRACTS, vol. 110, no. 9, 27.Februar 1989 Columbus, Ohio, US; abstract no. 75197t, A.A. GEVORKYAN ET AL. 'Anomalous intramolecular nucleophilic substitution in 1-acetoxy-4-halo-2-butenes under the action of strong bases' Seite 621; Spalte 2;
- ANGEWANDTE CHEMIE, Bd. 103, Nr. 10, Oktober 1991 WEINHEIM DE, Seiten 1376-1378, R. SCHWESINGER ET AL. 'Stabile Phosphazenium-Ionen in der Synthese - ein leicht zugängliches, extrem reaktives "nacktes" Fluoridsalz'
- ANGEWANDTE CHEMIE, Bd. 105, Nr. 9, September 1991 WEINHEIM DE, Seiten 1420-1422, R. SCHWESINGER ET AL. 'Wie stark und wie gehindert können ungeladene Phosphazenbasen sein?'

## Beschreibung

Die vorliegenden Erfindung betrifft ein Verfahren zur Herstellung von 2,5-Dihydrofuranen der allgemeinen Formel I 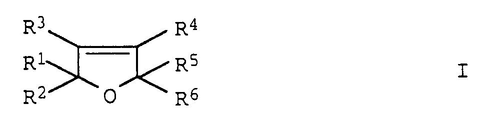 in der die Reste R¹, R², R³, R⁴, R⁵ und R⁶ gleich oder verschieden sind und für Wasserstoff oder C₁- bis C₄-Alkylgruppen stehen, durch die katalytische Umlagerung von 3,4-Epoxi-1-butenen der allgemeinen Formel II 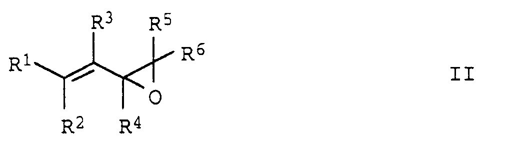 in Gegenwart einer Lewis-Säure oder elementaren Iods sowie in An- oder Abwesenheit eines Solubilisierungsmittels bei einer Temperatur von 60 bis 200°C.

2,5-Dihydrofuran ist nach US-A 3 812 158 durch die Isomerisierung von Vinyloxiran unter der Einwirkung von Quecksilbersalzen erhältlich. Abgesehen davon, daß bei diesem Verfahren 2,5-Dihydrofuran nur in maximal 33 % Ausbeute gewonnen werden kann, steht der industriellen Verwertung dieses Verfahrens die Verwendung der toxischen Quecksilbersalzkatalysatoren entgegen.

US-A 3 932 468 beschreibt die durch Übergangsmetallkomplexe und Brom- oder Iodwasserstoffgas katalysierte Umlagerung von Butadienmonoxid zu 2,5-Dihydrofuran. US-A 3 996 248 benötigt für diese Umlagerung ebenfalls Brom- oder Iodwasserstoff. Zur Verbesserung der Halogenwasserstoffkatalyse werden laut dieser Patentschrift homogen im Reaktionsmedium gelöste Lewis-Säuren, wie Zink-, Aluminium-, Bor-, Zinn- oder Magnesiumverbindungen, verwendet. Weiterhin wird dem Reaktionssystem Kaliumiodid zugesetzt.

Nachteilig an diesen beiden Verfahren ist, daß infolge der Anwesenheit von Halogenwasserstoffen im Reaktionsmedium bei der destillativen Isolierung des 2,5-Dihydrofurans erhebliche Verluste auftreten und das erhaltene Dihydrofuran durch Halogenwasserstoff und/oder Iod verunreinigt ist. So fällt die Ausbeute an 2,5-Dihydrofuran nach den Angaben von US-A 3 932 468 von 78 % im Rohprodukt (bestimmt mittels Gaschromatographie) auf 58 % nach der Destillation des Produktes ab, wobei das Destillat nur einen Dihydrofurangehalt von 89 % hat. Dieses Verfahren ist daher unwirtschaftlich.

Weitere Gründe für die Unwirtschaftlichkeit dieser Verfahren sind die Korrosionsprobleme, welche durch die Verwendung von Halogenwasserstoffkatalysatoren verursacht werden, sowie der hohe Verbrauch an Lösungsmitteln und Katalysatoren, welche nicht zurückgeführt werden können.

WO 91/13882 beschreibt ein Gasphasenverfahren zur Isomerisierung von Epoxyalkenen zu 2,5-Dihydrofuranen. Als Katalysatoren dienen Mischungen aus Ammoniumiodiden oder Phosphoniumiodiden und Lewis-Säuren auf einem Trägermaterial. Da sich Oniumhalogenide bei Temperaturen oberhalb 130°C allmählich zersetzen und das Epoxid bei der Umsetzung allmählich hochsiedende Oligomere und Polymere bildet, die sich auf der Katalysatoroberfläche abscheiden und diese blockieren, nimmt die Katalysatoraktivität und damit der Umsatz des Epoxids nach kurzer Betriebszeit erheblich ab, so daß dieses Verfahren unwirtschaftlich ist.

Weiterhin wird in WO 91/13882 die Isomerisierung von Epoxyalkenen in flüssiger Phase beschrieben, wobei als Lewis-Säuren Organozinn- oder Organoantimonverbindungen in Kombination mit einem Tetraalkylammonium- oder Phosphoniumiodid verwendet werden. Der gleiche Katalysatortyp wird in WO 93/10111 und US-A 5 238 889 angewandt.

Bereits in EP-A 412 366 wurde über ein Verfahren zur Isomerisierung von Epoxyalkenen zu 2,5-Dihydrofuranen in flüssiger Phase berichtet, in dem als Katalysator eine Lewis-Säure in Gegenwart eines Alkalimetall- oder Oniumiodids oder elementaren Iods in An- oder Abwesenheit eines Solubilisierungsmittels eingesetzt wurde. Die besten Ergebnisse wurden dabei mit der Kombination Zinkhalogenid/Alkalimetalliodid erzielt.

Obwohl die zuletzt genannten Flüssigphaseverfahren gute Ausbeuten an 2,5-Dihydrofuranen bei der Isomerisierung von Epoxybutenen liefern, sind sie dennoch verbesserungswürdig. So bilden sich im Zuge der Umsetzung Oligomere und Polymere des Epoxybutens, die insbesondere die Abtrennung der Alkalimetall- und Oniumhalogenide aus der Reaktionsmischung und somit die Rückgewinnung des Katalysators erschweren. Außerdem müssen die Alkalimetall- und Oniumhalogenide in relativ großen Mengen verwendet werden, damit befriedigende Ausbeuten und Selektivitäten erzielt werden können.

Der vorliegenden Erfindung lag somit die Aufgabe zugrunde, ein Verfahren zur Isomerisierung von Epoxybutenen zu 2,5-Dihydrofuranen zu finden, das frei von den vorgenannten Nachteilen ist. Insbesondere sollte ein Agens gefunden werden, das bei gleicher oder verbesserter Wirksamkeit die Funktion der Alkalimetall- oder Oniumhalogenide im oben geschilderten Katalysatorsystem wahrnehmen kann, das sich aus der Reaktionsmischung aber auf einfache Weise abtrennen läßt. Zudem sollte dieses Agens es ermöglichen, diese Umsetzung in Gegenwart geringerer Mengen an Lewis-Säure auszuüben, als dies nach dem Stand der Technik möglich ist.

Dementsprechend wurde ein Verfahren zur Herstellung von 2,5-Dihydrofuranen der allgemeinen Formel I 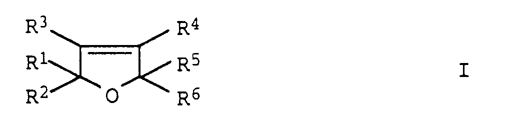 in der die Reste R¹, R², R³, R⁴, R⁵ und R⁶ gleich oder verschieden sind und für Wasserstoff oder C₁- bis C₄-Alkylgruppen stehen, durch die katalytische Umlagerung von 3,4-Epoxi-1-butenen der allgemeinen Formel II 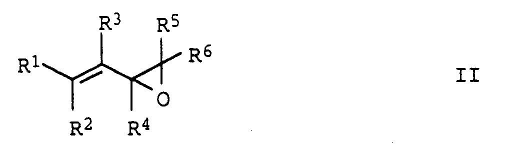 in Gegenwart einer Lewis-Säure oder elementaren Iods sowie in An- oder Abwesenheit eines organischen Solubilisierungsmittels bei einer Temperatur von 60 bis 200°C gefunden, das dadurch gekennzeichnet ist, daß man die Umsetzung in Gegenwart eines Phosphazeniumhalogenids oder eines Phosphazaniumhalogenids oder in Gegenwart von Gemischen aus Phosphazenium- und Phosphazaniumhalogeniden durchführt.

Im erfindungsgemäßen Verfahren werden vorzugsweise solche Phosphazeniumhalogenide oder Phosphazaniumhalogenide verwendet, wie sie in den allgemeinen Formeln III und IV dargesellt sind.

In den Phosphazeniumhalogeniden der allgemeinen Formel III, 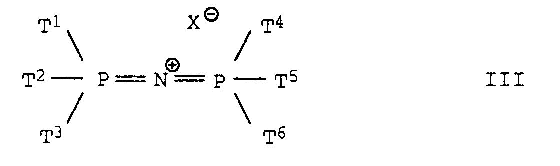 die auch als Phosphoranyliden-Iminiumhalogenide bezeichnet werden, ist X das Gegenanion zum positiv geladenen Stickstoffatom der Iminiumverbindung III. X ist im allgemeinen ein Chlorid-, Bromid- oder Iodid-Ion. Die Phosphoratome der Phosphazeniumverbindung III tragen insgesamt 6 Substituenten T¹, T², T³, T⁴, T⁵ und T⁶, die gleich oder verschieden sein können und unabhängig voneinander für gegebenenfalls substituierte C₁- bis C₂₀-, vorzugsweise C₁- bis C₁₀-, insbesondere C₁- bis C₆-Alkylgruppen, gegebenenfalls substituierte C₆- bis C₁₀-Arylgruppen, vorzugsweise die Phenyl- oder Naphthylgruppe, gegebenenfalls substituierte C₇- bis C₁₂-Aralkylgruppen, vorzugsweise die Benzyl-, Phenylethylen- oder Naphthylmethylen-Gruppe, Di-(C₁- bis C₁₀-alkyl-)amino-, vorzugsweise Di-(C₁- bis C₅-alkyl-)aminogruppen, insbesondere die Dimethylamino-, die Diethylamino-, Di-n-propylamino-, Di-i-propylamino-, Di-n-butylamino-, Di-i-butylamino-, Di-tert.butylamino-, Di-n-pentylamino- oder die Di-neopentylamino-Gruppe, Di-(C₃- bis C₈-cycloalkyl-)aminogruppen, vorzugsweise Di-(C₃- bis C₆-cycloalkyl-)aminogruppen, insbesondere Dicyclopropyl-, Dicyclopentyl- oder Dicyclohexyl-amino-Gruppen, Di-(C₆- bis C₁₀-aryl-)aminogruppen, vorzugsweise die Diphenylamino-Gruppe, (C₁- bis C₁₀-alkyl-) (C₆- bis C₁₀-aryl-)aminogruppen, beispielsweise die N-Methylanilino- oder die N-Methyl-naphthylamino-Gruppe, Di-(C₇- bis C₁₂-aralkyl-)aminogruppen, wie die Dibenzylamino-Gruppe, (C₁- bis C₁₀-alkyl) (C₇- bis C₁₂-aralkyl-)aminogruppen, wie die N,N-Methyl-benzyl-aminogruppe, (C₆- bis C₁₀-aryl-) (C₇- bis C₁₂-aralkyl-)aminogruppen, wie die N,N-Phenylbenzyl-amino-Gruppe, Pyrrolidinyl-, Piperidinyl-, Morpholinyl oder N'-(C₁- bis C₄-alkyl)-piperazinyl-Gruppen stehen.

Die Substituenten der Phosphoratome der Phosphazeniumhalogenide III T¹, T², T³, T⁴, T⁵ und T⁶ können, insbesondere wenn es sich dabei um aliphatische, aromatische oder araliphatische Kohlenwasserstoffgruppen handelt, gegebenenfalls 1 bis 3 weitere Substituenten tragen, welche sich unter den Reaktionsbedingungen des erfindungsgemäßen Verfahrens inert verhalten. Beispielhaft für solche Substituenten seien C₁- bis C₂₀-, vorzugsweise C₁- bis C₁₀-, insbesondere C₁ bis C₄-Alkoxygruppen oder Halogenatome, insbesondere Chlor- oder Bromatome genannt. Die aromatischen Ringe der Aryl- bzw. Aralkylsubstituenten können weiterhin als Substituenten C₁- bis C₂₀-, vorzugsweise C₁- bis C₁₀-, insbesondere C₁- bis C₄-Alkylsubstituenten tragen. Das Substitutionsmuster und der Substitutionsgrad kann in der Regel beliebig gewählt werden, da sich diese Substituenten im allgemeinen nicht kritisch auf die katalytische Wirksamkeit der betreffenden Phosphazeniumhalogenide III auswirken. Folglich werden vorzugsweise solche Phosphazeniumhalogenide III im erfindungsgemäßen Verfahren verwendet, deren Alkyl-, Aryl- bzw. Aralkyl-Substituenten T¹, T², T³, T⁴, T⁵ und T⁶ keine weiteren Substituenten tragen. Besonders bevorzugte Phosphazeniumhalogenide der allgemeinen Formel III mit gegebenenfalls substituierten Kohlenwasserstoffresten T¹, T², T³, T⁴, T⁵ und T⁶ sind solche, in denen diese Reste für unsubstituierte oder substituierte Arylgruppen stehen.

Die stickstoffhaltigen Substituenten der Phosphoratome T¹, T², T³, T⁴, T⁵ und T⁶, also die Dialkyl-, Dicycloalkyl-, Diaryl-, Diaralkyl-, Alkylaryl-, Alkylaralkyl- bzw. Arylaralkyl-aminogruppen, die Pyrrolidinyl-, Piperidinyl-, Morpholinyl- oder die N'-(C₁- bis C₄-alkyl)-piperazinyl-Gruppen sind über die freie Valenz ihres Stickstoffatoms an die einzelnen Phosphoratome der Phosphazeniumverbindung III gebunden.

Es können auch jeweils die zwei Reste der stickstoffhaltigen Substituenten T¹, T², T³, T⁴, T⁵ und T⁶, beispielsweise die Reste T¹ und T² oder T¹ und T² und T⁴ und T⁵ gemeinsam mit dem jeweiligen Phosphoratom einen Ring bilden, so daß beispielsweise mono- oder bicyclische Phosphazeniumsalze III vorliegen, wie sie beispielhaft formelmäßig durch die Formel 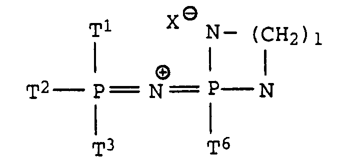 oder die Formel 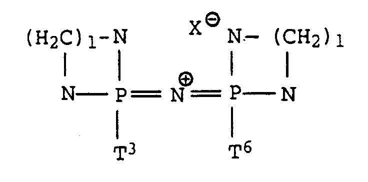 wiedergegeben werden können, in denen 1 eine ganze Zahl von 2 bis 5, vorzugsweise von 3 bis 4 bedeutet.

Es können auch jeweils drei der stickstoffhaltigen Reste T¹, T², T³ oder T⁴, T⁵, T⁶ jeweils gemeinsam mit dem betreffenden Phosphoratom einen bicyclischen Ring bilden, so daß sich Phosphazeniumalze III der Formel 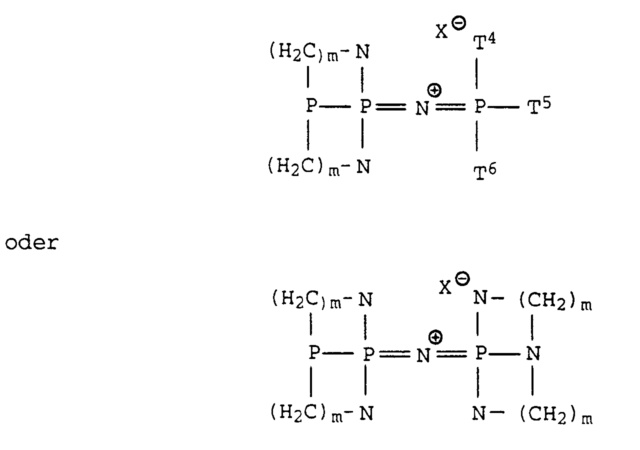 ergeben, in denen der Index m gleich oder verschieden sein kann und für eine ganze Zahl von 2 bis 5, vorzugsweise von 3 bis 4, steht.

Besonders bevorzugte Aminogruppen-Substituenten T¹, T², T³, T⁴, T⁵ und T⁶ sind Di-(C₁- bis C₁₀-alkyl-), insbesondere Di-(C₁- bis C₅-alkyl-)aminogruppen, wie die Dimethylamino-, die Diethylamino-, die Di-n-propylamino-, Diisopropylamino-, Di-n-butylamino-, Diisobutylamino-, Di-tert.butylamino-, Di-n-pentylamino-, Dineopentylamino-, Methyl-ethyl-amino-, Methyl-n-propyl-amino, Methyl-isopropyl-amino, Methyl-tert.butylamino-, Methyl-n-butylamino-, Methyl-neopentyl-amino-, Ethyl-n-propyl-amino-, Ethylisopropyl-amino-, Ethyl-n-butyl-amino-, Ethyl-isobutyl-amino- und die Ethyl-tert.butyl-amino-Gruppe, ferner die Diphenylamino-Gruppe, die Di-(C₃- bis C₆-cycloalkyl-)aminogruppen, insbesondere die Dicyclopropyl-, die Dicyclopentyl- sowie die Dicyclohexylaminogruppe sowie die cycloaliphatischen Aminogruppen, insbesondere die Pyrrolidinyl-, die Piperidinyl-, die Morpholinyl- sowie die N'-(C₁- bis C₄-alkyl)piperazinyl-Gruppen.

Die Reste T¹, T², T³, T⁴, T⁵ und T⁶ können, wie bereits erwähnt, unabhängig voneinander gleich oder verschieden sein, d.h., in den erfindungsgemäß anwendbaren Phosphazeniumhalogeniden III können die Substituenten T¹, T², T³, T⁴, T⁵ und T⁶ beispielsweise alle gleich sein und für eine bestimmte der obengenannten Substituenten-Gruppen stehen, es können die Phosphazeniumhalogenide III aber auch in einem Molekül verschiedenerlei Substituenten T¹, T², T³, T⁴, T⁵ und T⁶ enthalten, d.h. ein erfindungsgemäß anwendbares Phosphazeniumhalogenid der allgemeinen Formel III kann beispielsweise sowohl Kohlenwasserstoff- und Aminogruppen-haltige Substituenten T¹, T², T³, T⁴, T⁵ und T⁶ haben. Vorzugsweise werden aber solche Phosphazeniumhalogenide III im erfindungsgemäßen Verfahren eingesetzt, in denen die Reste T¹, T², T³, T⁴, T⁵ und T⁶ gleich oder zum überwiegenden Teil gleich sind.

Von der Klasse der Phospazeniumhalogenide der allgemeinen Formel III, in denen die Substituenten T¹, T², T³, T⁴, T⁵ und T⁶ für Kohlenwasserstoff-Substituenten stehen, sind die Phosphazeniumhalogenide der Formel IIIa 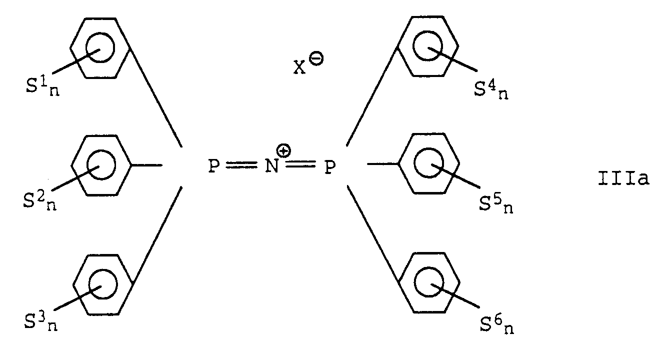 bevorzugt. Hierin ist X das Gegenanion zum positiv geladenen Stickstoffatom der Phosphazeniumverbindung. X ist im allgemeinen ein Halogenid-, beispielsweise ein Chlorid-, Bromid- oder Iodid-Ion. Die einzelnen Phenylringe der Phosphazeniumverbindung IIIa können unsubstituiert sein oder können 1 bis 3, vorzugsweise einen, der betreffenden Substituenten S¹, S², S³, S⁴, S⁵ bzw. S⁶ tragen. Die Substituenten S¹, S², S³, S⁴, S⁵ bzw. S⁶ können gleich oder verschieden sein und unabhängig voneinander für C₁- bis C₂₀-Alkyl, vorzugsweise C₁- bis C₁₀-Alkyl-, insbesondere C₁- bis C₄-Alkylgruppen, C₁- bis C₂₀-Alkoxy-, vorzugsweise C₁- bis C₁₀-Alkoxy-, insbesondere C₁- bis C₄-Alkoxygruppen oder Halogenatome, vorzugsweise Chlor- oder Bromatome, stehen. Dies bedeutet, daß jeder einzelne der 6 Phenylringe der Phosphazeniumverbindung IIIa unabhängig vom Substitutionsgrad und Substitutionsmuster der jeweils anderen Phenylringe in der Phosphazeniumverbindung IIIa entweder unsubstituiert sein kann oder 1 bis 3, vorzugsweise einen, gleiche oder verschiedene, vorzugsweise gleiche, der betreffenden Substituenten S¹, S², S³, S⁴, S⁵ bzw. S⁶ tragen kann. Der Index n bezeichnet dabei in Formel IIIa die Anzahl der betreffenden Substituenten S¹, S², S³, S⁴, S⁵ bzw. S⁶ am jeweiligen Phenylring und hat folglich einen Wert von 0, wenn der betreffende Phenylring unsubstituiert ist, oder einen ganzzahligen Wert von 1 bis 3, falls der betreffende Phenylring 1, 2 oder 3 der betreffenden Substituenten S¹, S², S³, S⁴, S⁵ bzw. S⁶ trägt. Die Substituenten S¹, S², S³, S⁴, S⁵ bzw. S⁶ sind dabei den jeweiligen Phenylringen zugeordnet, d.h., S¹ steht für den oder die Substituenten an Phenylring 1, S² für den oder die Substituenten an Phenylring 2 usw.

Der Substitutionsgrad und das Substitutionsmuster der einzelnen Phenylringe der Phosphazeniumverbindungen IIIa mit den betreffenden Substituenten S¹, S², S³, S⁴, S⁵ bzw. S⁶ ist im allgemeinen für die Aktivität der Phosphazeniumhalogenide IIIa als Cokatalysator im erfindungsgemäß angewandten Katalysatorsystem nicht kritisch. Aus diesem Grunde werden vorzugsweise solche Phosphazeniumhalogenide IIIa im erfindungsgemäßen Verfahren eingesetzt, die sich mit geringem Aufwand herstellen lassen. Beispiele für solche Phosphazeniumhalogenide IIIa sind im Folgenden genannt:

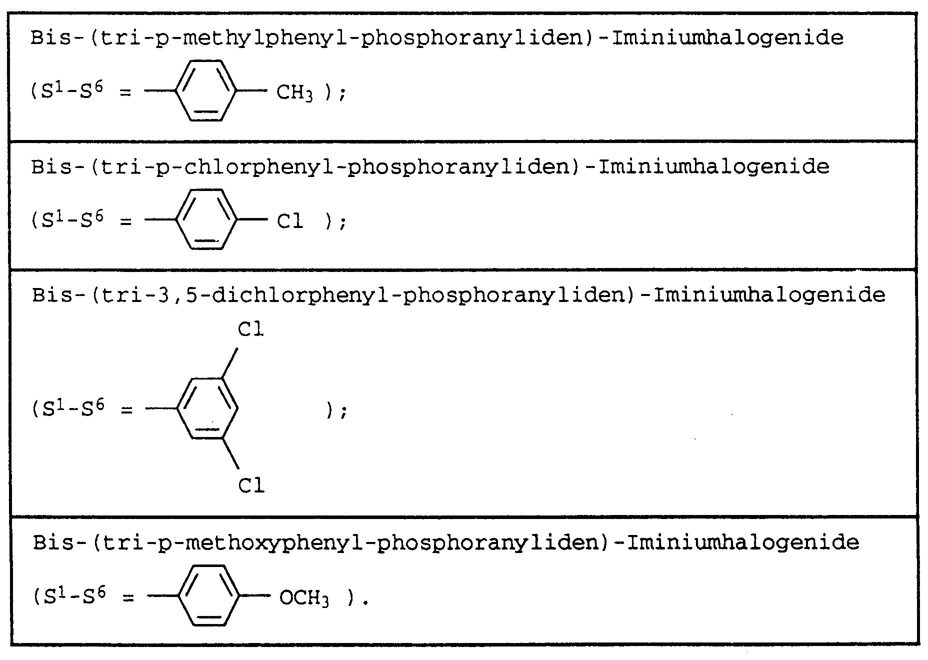

Besonders bevorzugt werden im erfindungsgemäßen Verfahren die unsubstituierten Bis-triphenylphosphazeniumhalogenide der Formel IIIb 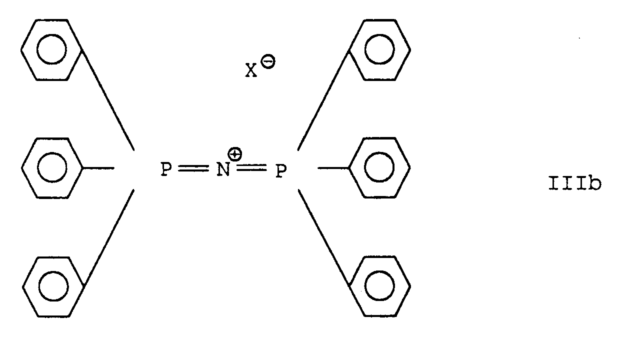 verwendet, worin X ein Chlorid-, Bromid- oder Iodid-Ion sein kann. Selbstverständlich können auch Gemische verschiedener Phosphazeniumhalogenide der allgemeinen Formel III im erfindungsgemäßen Verfahren eingesetzt werden, vorzugsweise wird aber ein individuelles Phosphazeniumsalz III verwendet.

Die Phosphazeniumsalze der allgemeinen Formel IIIa und insbesondere IIIb haben den Vorteil, daß sie auf sehr einfache Weise z.B. nach den Methoden von Martinsen et al., Acta Chem. Scand. Ser. A 31, 645 (1977), Kukushkin et al., Inorg. Chim. Acta 176, 79 (1990) oder Ruff et al., Inorganic Syntheses (Ed. G.W. Parshall), Vol. XV, S. 84 bis 87, Mc Graw-Hill, Hew York 1974 kostengünstig aus einfachen und gut verfügbaren Ausgangsmaterialien hergestellt werden können.

Von der Klasse der Phospazeniumhalogenide der allgemeinen Formel III, in denen die Substituenten T¹, T², T³, T⁴, T⁵ und T⁶ für Aminogruppen-Substituenten stehen, sind die Bis-[tris(dialkylamino)]-phosphazeniumhalogenide der allgemeinen Formel IIIc 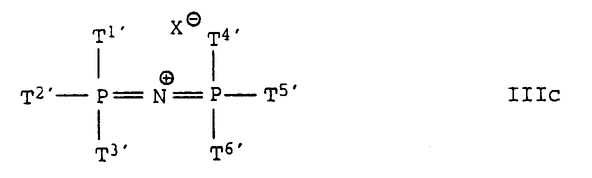 bevorzugt, in der die Substituenten T^{1'}, T^{2'}, T^{3'}, T^{4'}, T^{5'} und T^{6'} unabhängig voneinander für gleiche oder verschiedene, vorzugsweise gleiche, Di-(C₁- bis C₄-alkyl-)amino-Gruppen, Di-(C₃- bis C₆-cycloalkyl-)amino-Gruppen oder für cyclische Aminogruppenhaltige Substituenten, insbesondere Pyrrolidinyl-, Piperidinyl-, Morpholinyl- oder N'-(C₁- bis C₄-alkyl)-piperazinyl-Gruppen stehen, und X ein Chlorid-, Bromid- oder Iodid-Ion ist. Als Beispiele für besonders bevorzugte Phosphazeniumhalogenide des Typs IIIc seien genannt:
Bis[tris(dimethylamino)phosphoranyliden]iminiumhalogenide;
Bis[tris(N-pyrrolidinyl)phosphoranyliden]iminiumhalogenide;
Bis[tris(N-piperidinyl)phosphoranyliden]iminiumhalogenide;
Bis[tris(N-morpholinyl)phosphoranyliden]iminiumhalogenide;
Bis[tris(N'-methyl)-N-piperazinyl)phosphoranyliden]iminiumhalogenide;
Bis[tris(dicyclopropylamino)phosphoranyliden]iminiumhalogenide;
Bis[tris(dicyclopentylamino)phosphoranyliden]iminiumhalogenide;
Bis[tris(dicyclohexylamino)phosphoranyliden]iminiumhalogenide.

Im erfindungsgemäßen Verfahren können anstelle der Phosphazeniumhalogenide III oder gemeinsam mit diesen Phosphazaniumhalogenide der allgemeinen Formel IV 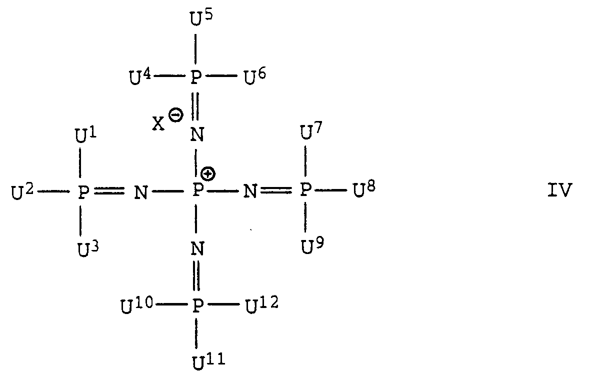 als Cokatalysatoren verwendet werden, in der die Substituenten U¹, U², U³, U⁴, U⁵, U⁶, U⁷, U⁸, U⁹, U¹⁰, U¹¹ und U¹² gleich oder verschieden sind und unabhängig voneinander für gegebenenfalls substituierte C₁- bis C₂₀ Alkylgruppen, gegebenenfalls substituierte C₆- bis C₁₀-Arylgruppen, gegebenenfalls substituierte C₇- bis C₁₂-Aralkylgruppen, Di-(C₁- bis C₁₀-alkyl-)aminogruppen, Di-(C₃- bis C₈-cycloalkyl-)aminogruppen, Di-(C₆- bis C₁₀-aryl-)aminogruppen, (C₁- bis C₁₀-alkyl-) (C₆- bis C₁₀-aryl-)aminogruppen, Di-(C₇- bis C₁₂-aralkyl-)aminogruppen, (C₁- bis C₁₀-alkyl-) (C₇- bis C₁₂-aralkyl-)aminogruppen, (C₆- bis C₁₀-aryl-) (C₇- bis C₁₂-aralkyl-)aminogruppen, Pyrrolidinyl-, Piperidinyl-, Morpholinyl- oder N'-(C₁- bis C₄-alkyl)-piperazinyl-Gruppen stehen. Bevorzugte Phosphazaniumsalze IV sind solche, in denen die Substituenten U¹, U², U³, U⁴, U⁵, U⁶, U⁷, U⁸, U⁹, U¹⁰, U¹¹ und U¹² unabhängig voneinander für gleiche oder verschiedene, vorzugswise gleiche, Di-(C₁- bis C₄-alkyl-)aminogruppen, Di-(C₃- bis C₆-cycloalkyl-)aminogruppen oder für cyclische Aminogruppen, insbesondere Pyrrolidinyl-, Piperidinyl-, Morpholinyl- oder N'-(C₁- bis C₄-alkyl)-piperazinyl-Gruppen stehen. In den Phosphazaniumhalogeniden IV ist X vorzugsweise ein Chlorid-, Bromid oder Iodid-Ion.

Die Phosphazaniumhalogenide IV können sowohl alleine oder in Mischungen mit den Phosphazeniumhalogeniden III als Cokatalysatoren im erfindungsgemäßen Verfahren eingesetzt werden. Vorzugsweise werden Phosphazaniumhalogenide IV alleine als Cokatalysatoren verwendet.

Die Phosphazeniumhalogenide des Typs gemäß Formel IIIc und die Phosphazaniumhalogenide IV lassen sich beispielsweise auf einfache Weise nach dem Baukastenprinzip aus den entsprechenden Bausteinen nach den allgemein anwendbaren Verfahren von Schwesinger et al., Angew. Chem. 99, 1212 (1987), Schwesinger et al., Angew. Chem. 103, 1376 (1991), Schwesinger et al., Angew. Chem. 104, 864 (1992) und Angew. Chem. 105, 1420 (1993) herstellen.

Als zweite Komponente des erfindungsgemäß anzuwendenden Katalysatorsystems dient eine Lewis-Säure. Eine Reihe solcher Lewis-Säuren, die als Katalysatoren für die Isomerisierung von Epoxybutenen zu 2,5-Dihydrofuranen in flüssiger Phase geeignet sind, wurde in EP-A 412 366, WO 91/13882, WO 93/10111 und US-A 5 238 889 genannt, weshalb hier auf diesen Stand der Technik beispielhaft verwiesen wird. Als Lewis-Säuren können z.B. insbesondere die üblicherweise in der präparativen, organischen Chemie verwendeten Lewis-Säuren, wie Zink-, Zirkonium-, Titan-, Nickel- oder Zinnhalogenide, verwendet werden. Zwar werden im erfindungsgemäßen Verfahren die Lewis-Säuren vorzugsweise in Form ihrer Halogenide eingesetzt, doch ist auch die Verwendung von halogenfreien Lewis-Säuren, z.B. Dialkylzinnoxiden, möglich. Die halogenhaltigen Lewis-Säuren können sowohl in Form der Metall-Halogen-Elementverbindungen, wie Zinkchlorid, Zinkbromid, Zinkiodid, Titantetrachlorid als auch in Form von Organometallhalogeniden, wie Trialkyl- oder Triaryl-Zinnhalogeniden, beispielsweise Triphenylzinniodid, Tributylzinniodid oder Tri-n-octylzinniodid, oder Tetraalkyl- oder Tetraaryl-Antimonhalogeniden, wie Tetraphenylstiboniumiodid, eingesetzt werden. Besonders bevorzugt werden im erfindungsgemäßen Verfahren die Lewis-Säuren Zinkchlorid, Zinkbromid, Zinkiodid, Triphenylzinniodid, Tributylzinniodid oder Tri-n-octylzinniodid benutzt. Es können selbstverständlich auch Gemische verschiedener Lewis-Säuren im erfindungsgemäßen Verfahren eingesetzt werden.

In der Regel erweist es sich für das erfindungsgemäße Verfahren als vorteilhaft, wenn wenigstens eine Komponente der Katalysatorkombination Phosphazeniumhalogenid III und/oder Phosphazaniumhalogenid IV/Lewis-Säure ein Iodid ist.

Anstelle der Lewis-Säure kann auch elementares Iod im erfindungsgemäßen Verfahren eingesetzt werden. Vermutlich wirkt das Iod in diesem Falle ähnlich den Lewis-Säuren als Elektronenakzeptor, doch ist dies, da das Schicksal des Iodmoleküls im Zuge der Umsetzung nicht verfolgt wurde, eine bloße Vermutung und schließt eine andere Reaktionsweise des Iods nicht aus.

Die Isomerisierung der Epoxybutene II zu den 2,5-Dihydrofuranen I mit Hilfe des erfindungsgemäßen Katalysatorsystems wird zweckmäßigerweise in Gegenwart eines Solubilisierungsmittels vorgenommen, das die Aufgabe hat, die Katalysatorkomponenten Phosphazeniumhalogenid III und/oder Phosphazaniumhalogenid IV und Lewis-Säure zu solubilisieren, d.h. sowohl den Kontakt und das katalytische Zusammenwirken dieser Komponenten als auch den Kontakt dieser Katalysatorkomponenten mit dem Reaktionsmedium zu fördern. Unter Reaktionsmedium wird hierbei insbesondere das Epoxybuten II sowie dessen Mischungen mit dem Dihydrofuran I und gegebenenfalls mit Oligomeren und Polymeren des Epoxybutens II, wie sie im Zuge der Umsetzung entstehen, verstanden. Als Solubilisierungsmittel eignen sich somit Substanzen, die in der Lage sind, die beiden Katalysatorkomponenten zu lösen oder auch nur anzulösen und auf diese Weise den Kontakt zwischen den Katalysatorkomponenten selbst als auch mit dem Reaktionsmedium zu verstärken. Da an das Solubilisierungsmittel, außer den Eigenschaften, die Komponenten des Katalysatorsystems, Phosphazeniumhalogenid III und/oder Phosphazaniumhalogenid IV und Lewis-Säure, zu solubilisieren und sich ansonsten unter den Reaktionsbedingungen inert zu verhalten, d.h. die Ausbeute und Selektivität der Umsetzung nicht nachteilig zu beeinflussen, im Prinzip keine weiteren Anforderungen bezüglich ihrer chemischen Eigenschaften gestellt werden müssen, können als Solubilisierungsmittel eine Vielzahl von Substanzen verwendet werden. Beispielsweise seien als Solubilisierungsmittel dipolar-aprotische Lösungsmittel, wie Tetramethylharnstoff, cyclische Harnstoffe, wie N,N-Dimethylethylenharnstoff oder N,N-Dimethylpropylenharnstoff, Phosphorsäuretriamide, wie Hexamethylphosphorsäuretriamid, N-Alkylpyrrolidone, wie N-Methylpyrrolidon, N-Octylpyrrolidon, N-Cyclohexylpyrrolidon, Amide, wie N,N-Dimethylformamid, N,N-Dimethylacetamid, N,N-Diethylacetamid, Ether, wie Tetrahydrofuran, Dioxan, Dimethoxyethan oder Polyethylenglykoldialkylether, Sulfoxide, wie Dimethylsulfoxid, oder Sulfone, wie Dimethylsulfon oder Sulfolan (Tetrahydrothiophen-1,1-dioxid) genannt.

Selbstverständlich können auch mehrere der genannten Solubilisierungsmittel nacheinander oder in Mischungen zur Solubilisierung der Katalysatorkomponenten Phosphazeniumhalogenid III und/oder Phosphazaniumhalogenid IV und Lewis-Säure zum Reaktionsansatz gegeben werden. Des weiteren können auch noch andere unter den Reaktionsbedingungen inerte Lösungsmittel, wie Diethylether, Ester, wie Ethylacetat, Ketone, wie Aceton oder aromatische Lösungsmittel, wie Benzol, Toluol oder Xylol zur Verdünnung des Reaktionsgemisches zugesetzt werden.

Da die zur Solubilisierung des Katalysatorsystems Phosphazeniumhalogenid III und/oder Phosphazaniumhalogenid IV/Lewis-Säure notwendige Menge des Solubilisierungsmittels im allgemeinen von der Art der verwendeten Lewis-Säure und vom Solubilisierungsmittel abhängt, empfiehlt es sich, die optimale Menge an zuzusetzendem Solubilisierungsmittel für das betreffende Solubilisierungsmittel in einem einfachen Vorversuch zu ermitteln.

Da die Phosphazeniumhalogenide III als auch die Phosphazaniumhalogenide IV selbst eine gewisse Löslichkeit im organischen Reaktionsmedium des Verfahrens, also im Epoxybuten II selbst sowie in den als Folge der Umsetzung gebildeten Gemischen aus Epoxybuten II, 2,5-Dihydrofuran I und Epoxybuten-Oligomeren und -Polymeren, haben und manche der Lewis-Säuren, insbesondere organometallische Lewis-Säuren, wie Dialkylzinnoxide, Trialkyl- oder Triarylzinnhalogenide oder Tetraalkylstiboniumhalogenide, ebenfalls inhärent eine gewisse Löslichkeit in diesem Reaktionsmedium haben, kann die Löslichkeit des Katalysatorsystems im organischen Reaktionsmedium so groß sein, daß der Zusatz eines Solubilisierungsmittels zum Reaktionsansatz nicht erforderlich ist. Dies gilt insbesondere, falls lipophile Phosphazeniumhalogenide III und/oder lipophile Phosphazaniumhalogenide IV verwendet werden, und mit diesen lipophilen Phosphazeniumhalogeniden III und/oder lipophilen Phosphazaniumhalogeniden IV im organischen Reaktionsmedium lösliche, organometallische Lewis-Säuren, wie die zuvor genannten, als Katalysatorsystem eingesetzt werden. Gewünschtenfalls kann auch eine Mischung aus Epoxybuten II mit dem Reaktionsprodukt 2,5-Dihydrofuran sowie den im Zuge der Umsetzung gebildeten Epoxybuten-Oligomeren und -Polymeren von Anfang an in die Umsetzung eingesetzt werden.

Die Katalysatorkomponenten Phosphazeniumhalogenid III und/oder Phosphazaniumhalogenid IV/Lewis-Säure werden zur Durchführung der Umsetzung üblicherweise in An- oder Abwesenheit des Solubilisierungsmittels im Reaktor vorgelegt und dazu das umzusetzende Epoxybuten II gepumpt. Diese Zugabereihenfolge ist jedoch nicht kritisch und kann umgestellt werden. Das Solubilisierungsmittel wird zweckmäßigerweise der Reaktionsmischung in solchen Mengen zugesetzt, daß der größte Teil des Katalysatorsystems Phosphazeniumhalogenid III und/oder Phosphazaniumhalogenid IV/Lewis-Säure in Lösung geht.

Das erfindungsgemäße Verfahren kann diskontinuierlich, z.B. in Rührkesseln oder kontinuierlich z.B. in Kaskaden-, Rohr- oder Schlaufenreaktoren ausgeübt werden.

Bei der diskontinuierlichen Betriebsweise wird das Phosphazeniumhalogenid III und/oder Phosphazaniumhalogenid IV, bezogen auf eingesetztes Butadienepoxid II in der Regel in Mengen von 0,01 bis 5 Gew.-%, vorzugsweise von 0,5 bis 1 Gew.-%, und die Lewis-Säure in Mengen von 0,02 bis 10 Gew.-%, vorzugsweise von 0,5 bis 2 Gew.-%, eingesetzt.

Da das erfindungsgemäße Katalysatorsystem echt katalytisch wirkt, d.h. bei der Isomerisierung des Epoxybutens II zum 2,5-Dihydrofuran I praktisch nicht verbraucht wird, sind die obengenannten Konzentrationsangaben nur als Orientierungswerte für das diskontinuierliche Verfahren anzusehen.

Dementsprechend wird bei der kontinuierlichen Verfahrensweise im allgemeinen eine bestimmte Menge des Katalysatorsystems im Reaktor vorgelegt, das Epoxybuten kontinuierlich zugeführt und das 2,5-Dihydrofuran laufend, in dem Maße wie es gebildet wird, beispielsweise destillativ, aus der Reaktionsmischung entfernt und seiner weiteren Verwertung zugeführt. Bei Verwendung von Rohrreaktoren im kontinuierlichen Verfahren wird die nach Abtrennung des 2,5-Dihydrofuran-Produktes erhaltene Lösung des Katalysatorsystems im Solubilisierungsmittel zweckmäßigerweise wieder in den Reaktor zurückgeleitet, d.h. die Katalysatorlösung wird im Kreis geführt. Es kann auch ein Teilstrom der Katalysatorlösung im Kreis geführt werden. Analog zum kontinuierlichen Verfahren kann im diskontinuierlichen Verfahren die nach Abtrennung des 2,5-Dihydrofurans zurückgebliebene Katalysatorlösung erneut zur Katalyse der Epoxybuten-Isomerisierung eingesetzt werden. Das Phosphazeniumhalogenid III und/oder das Phosphazaniumhalogenid IV wird im allgemeinen bezüglich der Lewis-Säure in einem Gewichtsverhältnis Phosphazeniumhalogenid III und/oder Phosphazaniumhalogenid IV/Lewis-Säure von 1:0,01 bis 1:10, vorzugsweise von 1:0,02 bis 1:2, zugesetzt.

Das erfindungsgemäße Verfahren wird im allgemeinen bei Temperaturen von 60 bis 200°C, vorzugsweise bei 90 bis 180°C, durchgeführt. Je nach Art und Menge des verwendeten Katalysatorsystems und in Abhängigkeit von der Reaktionstemperatur werden im allgemeinen Reaktionszeiten von wenigen Minuten bis zu 24 Stunden für eine vollständige Umsetzung benötigt.

Im allgemeinen wird das erfindungsgemäße Verfahren bei Atmosphärendruck ausgeübt. Die Durchführung des Verfahrens bei vermindertem Druck oder bei erhöhtem Druck, insbesondere unter Eigendruck, ist ebenfalls möglich.

Ein besonderer Vorteil des erfindungsgemäßen Verfahrens besteht darin, daß die Phosphazeniumhalogenide III als auch die Phosphazaniumhalogenide IV von oligomeren und polymeren Nebenprodukten, die sich bei längerem Betrieb in der Katalysatormischung anreichern, leicht dadurch abgetrennt werden können, daß man die Phosphazeniumhalogenide III bzw. Phosphazaniumhalogenide IV durch Abkühlen aus der Katalysatormischung auskristallisieren läßt und durch Filtration isoliert. Die Kristallisation kann durch Zusatz eines Lösungsmittels wie Toluol, Methanol, Ethanol, Aceton oder Essigester gefördert werden. Die auf diese Weise zurückgewonnenen Phosphazeniumhalogenide III bzw. Phosphazaniumhalogenide IV können anschließend wieder als Katalysatorkomponente verwendet werden. Diese Regenerierung der Katalysatorlösung kann sowohl diskontinuierlich oder kontinuierlich erfolgen, beispielsweise indem aus der rückzuführenden Katalysatorlösung ein Teilstrom zum Zwecke der Regenerierung abgezweigt, auf die geschilderte Weise regeneriert und anschließend wieder in die Umsetzung zurückgeführt wird.

Wegen der guten Wirksamkeit der Phosphazeniumhalogenide III als auch der Phosphazaniumhalogenide IV als Cokatalysatoren werden zur Erzielung eines gleich guten Ergebnisses bei der Isomerisierung von Epoxybutenen II zu 2,5-Dihydrofuranen I im allgemeinen geringere Mengen dieser Katalysatorkomponenten benötigt als bei Einsatz von Alkalimetall-, Ammonium- oder Phosphoniumhalogeniden, die bislang für diesen Zweck verwendet werden. Desgleichen kann aufgrund der guten Wirksamkeit der Phosphazeniumhalogenide III als auch der Phosphazaniumhalogenide IV der Gehalt der Lewis-Säure im erfindungsgemäßen Katalysatorsystem erniedrigt werden. Beispielsweise können bereits bei einem Lewis-Säure-Gehalt des Katalysatorsystems von 5 Mol-%, bezogen auf das Phosphazeniumhalogenid IIIb, gute Ergebnisse erzielt werden.

Vorteilhaft ist auch, daß besonders preiswerte Lewis-Säuren, wie Zinkhalogenide, in Kombination mit den Phosphazeniumhalogeniden III als auch mit den Phosphazaniumhalogeniden IV im erfindungsgemäßen Verfahren sehr gute Selektivitäten haben, so daß der Einsatz kostspieligerer organometallischer Lewis-Säuren, wie Organozinnhalogeniden, welche üblicherweise zwecks Erhöhung der Selektivität der Umsetzung in Kombination mit Phosphoniumiodiden verwendet werden, gewünschtenfalls unterbleiben kann.

Die als Ausgangsmaterialien benötigten Epoxybutene II sind beispielsweise nach der Methode von Kadesch (J. Am. Chem. Soc. 68, 41 (1946) oder nach den Verfahren von US-A 4 897 498 und US-A 4 950 773 erhältlich.

Die nach dem erfindungsgemäßen Verfahren erhältlichen 2,5-Dihydrofurane I können nach üblichen Verfahren zu den entsprechenden Tetrahydrofuranen hydriert werden, welche als Lösungsmittel sowie als Monomere zur Herstellung von Polytetrahydrofuranen dienen.

### Beispiele

### Beispiel 1

In einen 250-ml-Rührkolben wurde folgende Mischung eingefüllt und auf 130°C geheizt:
10 g Bis-(triphenylphosphoranyliden)-Iminiumiodid (IIIb),
0,7 g Zinkiodid und
60 g N-Cyclohexylpyrrolidon.

Mit einer mengengeregelten Pumpe wurden 32 g Vinyloxiran (Epoxybuten) pro Stunde zudosiert. Gleichzeitig destillierte dabei ein Gemisch von 2,5-Dihydrofuran und nicht umgesetztem Vinyloxiran ab.

Nach 10 h waren 320 g Vinyloxiran zugepumpt und 300 g Destillat erhalten worden. Nach Reduzierung des Drucks auf 10 mbar erhielt man weitere 14 g Destillat. Durch GC-Analyse wurde festgestellt, daß die vereinigten Destillate folgende Zusammensetzung hatten:
84 Gew.-% 2,5-Dihydrofuran
15,7 Gew.-% Vinyloxiran
0,3 Gew.-% Crotonaldehyd.

Bei 84,6 % Umsatz wurde damit eine Selektivität von 97,4 % erzielt.

Das Destillat wurde anschließend mit der gleichen Geschwindigkeit ein zweites Mal durch die Katalysatorlösung gefahren, wobei nach Ende des Zulaufs wieder der Druck reduziert wurde.

Dabei wurden 308 g Destillat erhalten mit der Zusammensetzung
97,7 Gew.-% 2,5-Dihydrofuran
2 Gew.-% Vinyloxiran
0,3 Gew.-% Crotonaldehyd.

Die Gesamtselektivität über beide Stufen betrug 96 % bei 98 % Umsatz.

Der nach dem Abdestillieren der Leichtsieder zurückbleibende flüssige Destillationsrückstand wurde mit 90 ml Essigsäureethylester versetzt, wobei ein Feststoff ausfiel. Nach 6 h wurde der Feststoff abfiltriert und die erhaltenen Kristalle mit Essigsäureethylester gewaschen und anschließend bei vermindertem Druck von Lösungsmittelresten getrocknet. Der so getrocknete Feststoff wurde gewogen und chemisch analysiert. Er bestand aus einer Mischung von 0,6 g Zinkiodid mit 9 g Bis-(triphenylphosphoranyliden)-Iminiumiodid. Diese Mischung konnte wieder als Katalysator in die Umsetzung eingesetzt werden.

### Beispiel 2

32,3 g Bis-(triphenylphosphoranyliden)-Iminiumiodid
21 g Triphenylzinniodid und
60 g Polyethylenglykoldimethylether (MW 2000)
wurden in einem Rührkolben auf 105°C geheizt. Während 10 h wurden gleichmäßig 510 g Vinyloxiran zudosiert und die Leichtsieder gleichzeitig abdestilliert. Nach Ende des Zulaufs wurde der Druck auf 10 mbar reduziert.

Es wurden 507 g Destillat mit der Zusammensetzung
20,1 Gew.-% Vinyloxiran
78,9 Gew.-% Dihydrofuran und
1 Gew.-% Crotonaldehyd
erhalten.

Daraus errechnet sich eine Selektivität von 98 % bei 80 % Umsatz.

### Beispiel 3

In einem 250-ml-Rührkolben wurde folgende Mischung eingefüllt und auf 130°C geheizt:
10 g Tetrakis-[tris(dimethylamino)phorphoranylidenimino]-Phosphazaniumiodid der Formel 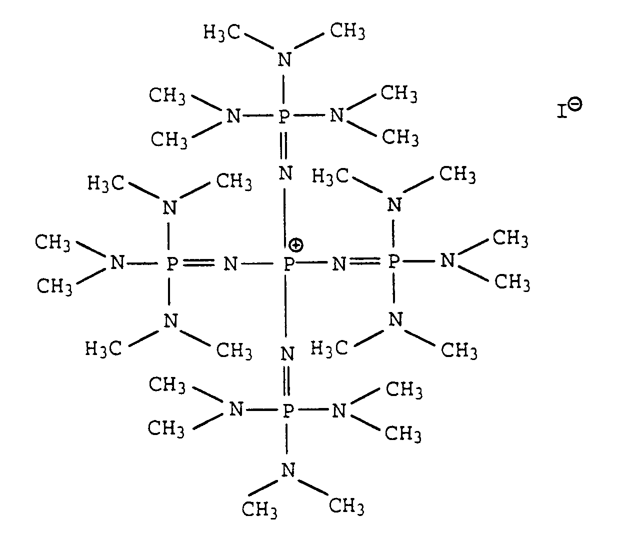
0,55 g Zinkiodid und
47 g N-Cyclohexyl-pyrrolidon.

Während 10 h wurden 400 g Vinyloxiran zudosiert und die Leichtsieder gleichzeitig abdestilliert. Nach Ende des Zulaufs wurde der Druck auf 10 mbar reduziert.

Es wurden 390 g Destillat mit der Zusammensetzung
28 Gew.-% Vinyloxiran,
71,5 Gew.-% 2,5-Dihydrofuran und
0,5 Gew.-% Crotonaldehyd
erhalten.

Daraus errechnet sich eine Selektivität von 96 % bei 73 % Umsatz.

### Beispiel 4

In einem 250-ml-Rührkolben wurde folgende Mischung eingefüllt und auf 130°C geheizt:
7 g Bis[tris(dimethylamino)phosphoranyliden]-Iminiumiodid der Formel 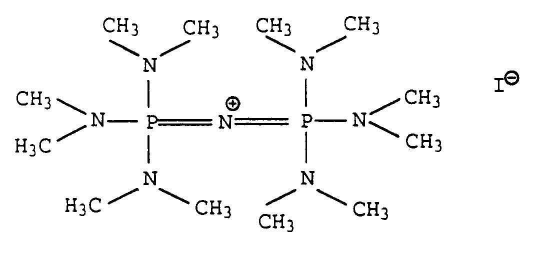
0,7 g Zinkiodid und
60 g N-Cyclohexyl-pyrrolidon.

Während 10 h wurden 510 g Vinyloxiran zudosiert und die Leichtsieder gleichzeitig abdestilliert. Nach Ende des Zulaufs wurde der Druck auf 10 mbar reduziert.

Es wurden 500 g Destillat mit der Zusammensetzung
18 Gew.-% Vinyloxiran,
81,6 Gew.-% 2,5-Dihydrofuran und
0,4 Gew.-% Crotonaldehyd
erhalten.

Daraus errechnet sich eine Selektivität von 98 % bei 82 % Umsatz.

## Patentansprüche

1. Verfahren zur Herstellung von 2,5-Dihydrofuranen der allgemeinen Formel I 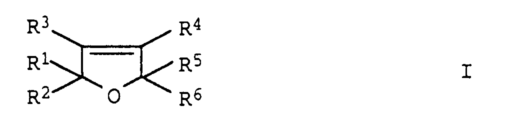 in der die Reste R¹, R², R³, R⁴, R⁵ und R⁶ gleich oder verschieden sind und für Wasserstoff oder C₁- bis C₄-Alkylgruppen stehen, durch die katalytische Umlagerung von 3,4-Epoxi-1-butenen der allgemeinen Formel II 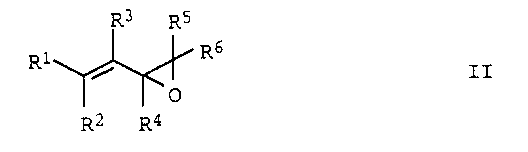 in Gegenwart einer Lewis-Säure oder elementaren Iods sowie in An- oder Abwesenheit eines Solubilisierungsmittels bei einer Temperatur von 60 bis 200°C, dadurch gekennzeichnet, daß man die Umsetzung in Gegenwart eines Phosphazeniumhalogenids oder eines Phosphazaniumhalogenids oder in Gegenwart von Gemischen aus Phosphazenium- und Phosphazaniumhalogeniden durchführt.

2. Verfahren nach Anspruch 1, dadurch gekennzeichnet, daß man die Umsetzung in Gegenwart eines Phosphazeniumhalogenids der allgemeinen Formel III 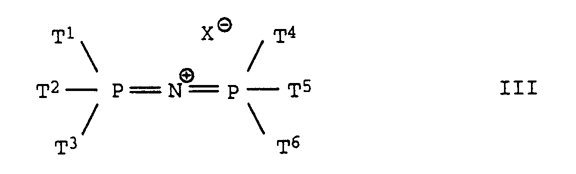 durchführt, worin X ein Chlorid-, Bromid- oder Iodid-Ion bedeutet und worin die Substituenten T¹, T², T³, T⁴, T⁵ und T⁶ gleich oder verschieden sind und unabhängig voneinander für gegebenenfalls substituierte C₁- bis C₂₀-Alkylgruppen, gegebenenfalls substituierte C₆- bis C₁₀-Arylgruppen, gegebenenfalls substituierte C₇- bis C₁₂-Aralkylgruppen, Di-(C₁- bis C₁₀-alkyl-)aminogruppen, Di-(C₃- bis C₈-cycloalkyl-)aminogruppen, Di-(C₆- bis C₁₀-aryl-)aminogruppen, (C₁- bis C₁₀-alkyl-) (C₆- bis C₁₀-aryl-)aminogruppen, Di-(C₇- bis C₁₂-aralkyl-)aminogruppen, (C₁- bis C₁₀-alkyl-) (C₇- bis C₁₂-aralkyl-)aminogruppen, (C₆- bis C₁₀-aryl-) (C₇- bis C₁₂-aralkyl-)aminogruppen, Pyrrolidinyl-, Piperidinyl-, Morpholinyl- oder N'-(C₁- bis C₄-alryl)-piperazinyl-Gruppen stehen.

3. Verfahren nach Anspruch 1 und 2, dadurch gekennzeichnet, daß man die Umsetzung in Gegenwart eines Phosphazeniumhalogenids der allgemeinen Formel IIIa 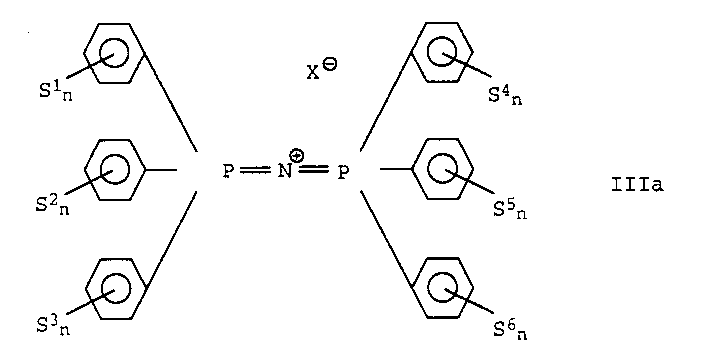 durchführt, worin X für ein Chlorid-, Bromid- oder Iodid-Ion steht und jeder einzelne der 6 Phenylringe des Phosphazeniummhalogenids IIIa entweder unsubstituiert ist oder 1 bis 3 gleiche oder verschiedene der betreffenden Substituenten S¹, S², S³, S⁴, S⁵ bzw. S⁶ trägt, wobei S¹, S², S³, S⁴, S⁵ bzw. S⁶ C₁- bis C₂₀-Alkylgruppen, C₁- bis C₂₀-Alkoxygruppen, Chlor und/oder Brom bedeuten und n die Anzahl der betreffenden Substituenten S¹, S², S³, S⁴, S⁵ bzw. S⁶ am jeweiligen Phenylring bezeichnet und einen ganzzahligen Wert von 0 bis 3 hat.

4. Verfahren nach den Ansprüchen 1 bis 3, dadurch gekennzeichnet, daß man die Umsetzung in Gegenwart eines Phosphazeniumhalogenids der Formel IIIb 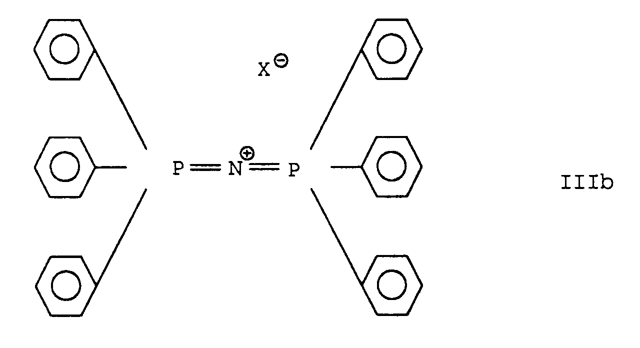 durchführt, in der X für ein Chlorid-, Bromid- oder Iodid-Ion steht.

5. Verfahren nach Anspruch 1 und 2, dadurch gekennzeichnet, daß man die Umsetzung in Gegenwart eines Phosphazeniumhalogenids der Formel IIIc 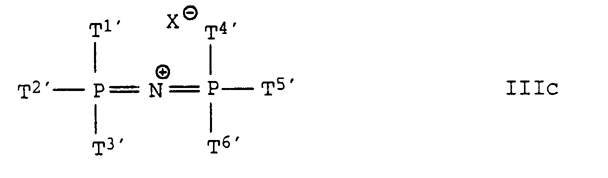 durchführt, in der die Reste T^{1'}, T^{2'}, T^{3'}, T^{4'}, T^{5'} und T⁶' gleich oder verschieden sind und unabhängig voneinander für Di-(C₁- bis C₄-alkyl-)aminogruppen oder für Pyrrolidinyl-, Piperidinyl-. Morpholinyl- oder N'-(C₁- bis C₄-alkyl)-piperazinyl-Gruppen stehen und X ein Chlorid-, Bromid- oder Iodid-Ion ist.

6. Verfahren nach Anspruch 1, dadurch gekennzeichnet, daß man die Umsetzung in Gegenwart eines Phosphazaniumhalogenids der Formel IV 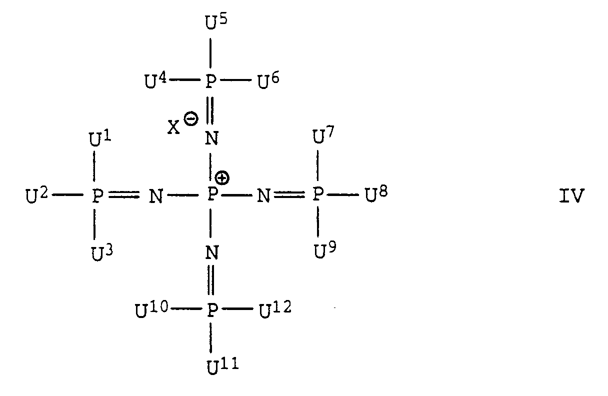 durchführt, worin die Substituenten U¹, U², U³, U⁴, U⁵, U⁶, U⁷, U⁸, U⁹, U¹⁰, U¹¹ und U¹² gleich oder verschieden sind und unabhängig voneinander für gegebenenfalls substituierte C₁- bis C₂₀-Alkylgruppen, gegebenenfalls substituierte C₆- bis C₁₀-Arylgruppen, gegebenenfalls substituierte C₇- bis C₁₂-Aralkylgruppen, Di-(C₁- bis C₁₀-alkyl-)aminogruppen, Di-(C₃- bis C₈-cycloalkyl-)aminogruppen, Di-(C₆- bis C₁₀-aryl-)aminogruppen, (C₁- bis C₁₀-alkyl-) (C₆- bis C₁₀-aryl-)aminogruppen, Di-(C₇- bis C₁₂-aralkyl-)aminogruppen, (C₁- bis C₁₀-alkyl-) (C₇- bis C₁₂-aralkyl-)aminogruppen, (C₆- bis C₁₀-aryl-) (C₇- bis C₁₂-aralkyl-)aminogruppen, Pyrrolidinyl-, Piperidinyl-, Morpholinyl- oder N'-(C₁- bis C₄-alkyl)-piperazinyl-Gruppen stehen.

7. Verfahren nach den Ansprüchen 1 und 6, dadurch gekennzeichnet, daß man die Umsetzung in Gegenwart eines Phosphazaniumhalogenids der Formel IV durchführt, worin die Substituenten U¹, U², U³, U⁴, U⁵, U⁶, U⁷, U⁸, U⁹, U¹⁰, U¹¹ und U¹² gleich oder verschieden sind und unabhängig voneinander für Di-(C₁- bis C₄-alkyl-)aminogruppen, Pyrrolidinyl-, Piperidinyl-, Morpholinyl- oder N'-(C₁- bis C₄-alkyl)-piperazinyl-Gruppen stehen und X ein Chlorid-, Bromid- oder Iodid-Ion ist.

8. Verfahren nach den Ansprüchen 1 bis 7, dadurch gekennzeichnet, daß man die Phosphazenium- und/oder Phosphazaniumhalogenide der allgemeinen Formeln III und IV bezüglich der Lewis-Säure in einem Gewichtsverhältnis Phosphazeniumhalogenid III und/oder Phosphazaniumhalogenid IV : Lewis-Säure von 1:0,01 bis 1:10 einsetzt.

9. Verfahren nach den Ansprüchen 1 bis 8, dadurch gekennzeichnet, daß wenigstens eine der Komponenten des verwendeten Katalysatorsystems Phosphazeniumhalogenid III und/oder Phosphazaniumhalogenid IV/Lewis-Säure ein Iodid ist.

10. Verfahren nach den Ansprüchen 1 bis 9, dadurch gekennzeichnet, daß man in Anwesenheit eines Solubilisierungsmittels arbeitet.

11. Verfahren nach den Ansprüchen 1 bis 9, dadurch gekennzeichnet, daß man in Abwesenheit eines Solubilisierungsmittels arbeitet.

12. Verfahren nach den Ansprüchen 1 bis 11, dadurch gekennzeichnet, daß man ein Zinkhalogenid als Lewis-Säure verwendet.

13. Verfahren nach den Ansprüchen 1 bis 11, dadurch gekennzeichnet, daß man eine organometallische Lewis-Säure verwendet.

14. Kontinuierliche Ausgestaltung des Verfahrens gemäß Anspruch 1, dadurch gekennzeichnet, daß man einer Mischung aus einem Phosphazeniumhalogenid oder Phosphazaniumhalogenid oder aus Phosphazeniumhalogenid/Phosphazaniumhalogenid-Mischungen mit einer Lewis-Säure in An- oder Abwesenheit eines organischen Solubilisierungsmittels ein Epoxybuten der allgemeinen Formel II bei Reaktionstemperatur zuführt und das bei der Umsetzung gebildete Dihydrofuran I noch während der Umsetzung, kontinuierlich abdestilliert.

15. Verfahren nach den Ansprüchen 1 bis 14, dadurch gekennzeichnet, daß man als Epoxybuten II Vinyloxiran der Formel IIa 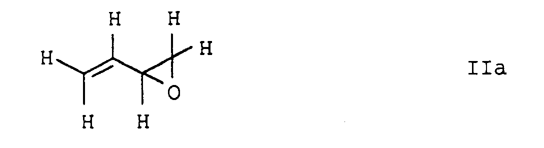 einsetzt.

## Claims

1. A process for preparing 2,5-dihydrofurans of the general formula I 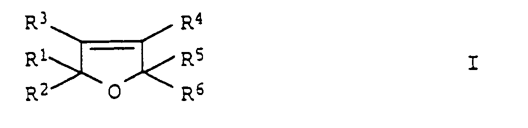 where the radicals R¹, R², R³, R⁴, R⁵ and R⁶ are identical or different and are hydrogen or C₁-C₄-alkyl groups, by catalytic rearrangement of 3,4-epoxy-1-butenes of the general formula II 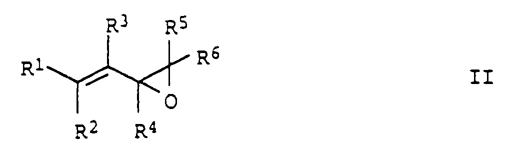 in the presence of a Lewis acid or elemental iodine and in the presence or absence of a solubilizer at from 60 to 200°C, wherein the reaction is carried out in the presence of a phosphazenium halide or of a phosphazanium halide or in the presence of mixtures of phosphazenium and phosphazanium halides.

2. A process as claimed in claim 1, wherein the reaction is carried out in the presence of a phosphazenium halide of the general formula III 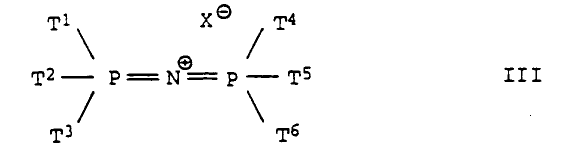 where X is a chloride, bromide or iodide ion, and where the substituents T¹, T², T³, T⁴, T⁵ and T⁶ are identical or different and are, independently of one another, unsubstituted or substituted C₁-C₂₀-alkyl groups, unsubstituted or substituted C₆-C₁₀-aryl groups, unsubstituted or substituted C₇-C₁₂-aralkyl groups, di-(C₁-C₁₀-alkyl)amino groups, di-(C₃-C₈-cycloalkyl)amino groups, di-(C₆-C₁₀-aryl)amino groups, (C₁-C₁₀-alkyl) (C₆-C₁₀-aryl)amino groups, di-(C₇-C₁₂-aralkyl)amino groups, (C₁-C₁₀-alkyl) (C₇-C₁₂-aralkyl)amino groups, (C₆-C₁₀-aryl) (C₇-C₁₂-aralkyl)amino groups, pyrrolidinyl, piperidinyl, morpholinyl or N'-(C₁-C₄-alkyl)piperazinyl groups.

3. A process as claimed in claim 1 and 2, wherein the reaction is carried out in the presence of a phosphazenium halide of the general formula IIIa 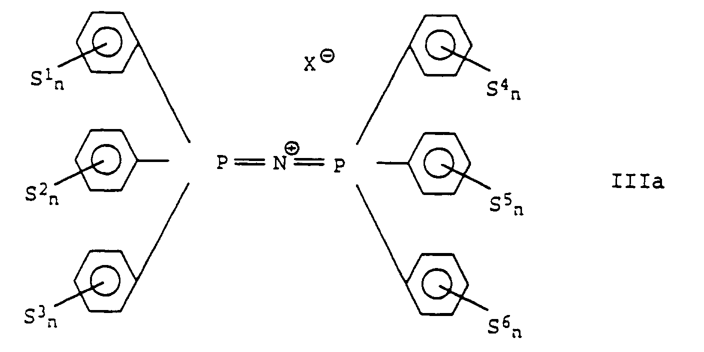 where X is a chloride, bromide or iodide ion and each one of the 6 phenyl rings in the phosphazenium halide IIIa either is unsubstituted or has 1 to 3 of the relevant substituents S¹, S², S³, S⁴, S⁵ and S⁶, which are identical or different and where S¹, S², S³, S⁴, S⁵ and S⁶ are C₁-C₂₀-alkyl groups, C₁-C₂₀-alkoxy groups, chlorine and/or bromine, and n is the number of the relevant substituents S¹, S², S³, S⁴, S⁵ and S⁶ on the particular phenyl ring and has an integral value from 0 to 3.

4. A process as claimed in claims 1 to 3, wherein the reaction is carried out in the presence of a phosphazenium halide of the formula IIIb 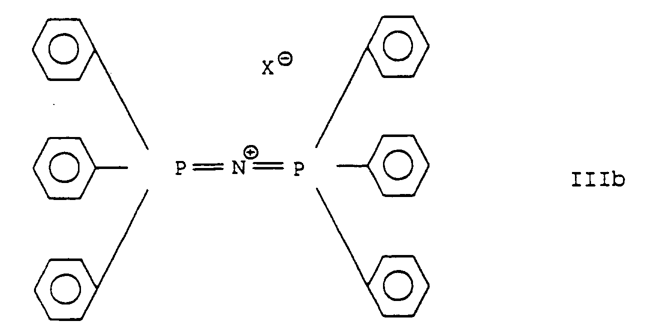 where X is a chloride, bromide or iodide ion.

5. A process as claimed in claim 1 and 2, wherein the reaction is carried out in the presence of a phosphazenium halide of the formula IIIc 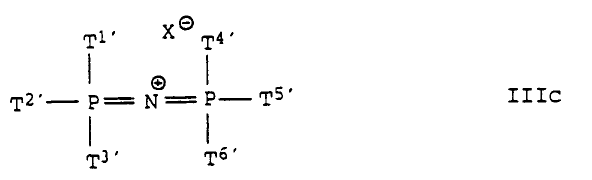 where the radicals T^{1'}, T^{2'}, T^{3'}, T^{4'}, T^{5'} and T^{6'} are identical or different and are, independently of one another, di-(C₁-C₄-alkyl)amino groups or pyrrolidinyl, piperidinyl, morpholinyl or N'-(C₁-C₄-alkyl)piperazinyl groups, and X is a chloride, bromide or iodide ion.

6. A process as claimed in claim 1, wherein the reaction is carried out in the presence of a phosphazanium halide of the formula IV 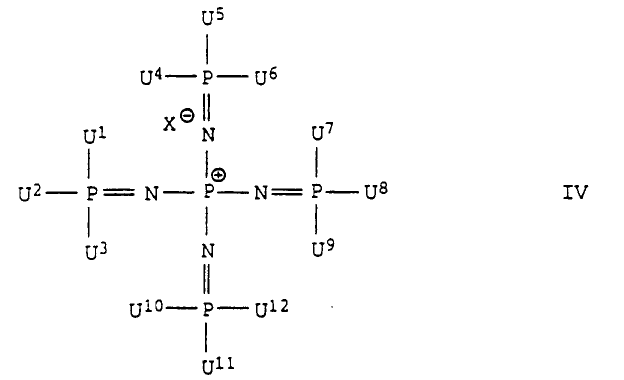 where the substituents U¹, U², U³, U⁴, U⁵, U⁶, U⁷, U⁸, U⁹, U¹⁰, U¹¹ and U¹² are identical or different and are, independently of one another, unsubstituted or substituted C₁-C₂₀-alkyl groups, unsubstituted or substituted C₆-C₁₀-aryl groups, unsubstituted or substituted C₇-C₁₂-aralkyl groups, di-(C₁-C₁₀-alkyl)amino groups, di-(C₃-C₈-cycloalkyl)amino groups, di-(C₆-C₁₀-aryl)amino groups, (C₁-C₁₀-alkyl) (C₆-C₁₀-aryl)amino groups, di-(C₇-C₁₂-aralkyl)amino groups, (C₁-C₁₀-alkyl) (C₇-C₁₂-aralkyl)amino groups, (C₆-C₁₀-aryl) (C₇-C₁₂-aralkyl)amino groups, pyrrolidinyl, piperidinyl, morpholinyl or N'-(C₁-C₄-alkyl)piperazinyl groups.

7. A process as claimed in claims 1 and 6, wherein the reaction is carried out in the presence of a phosphazanium halide of the formula IV where the substituents U¹, U², U³, U⁴, U⁵, U⁶, U⁷, U⁸, U⁹, U¹⁰, U¹¹ and U¹² are identical or different and are, independently of one another, di-(C₁-C₄-alkyl)amino groups, pyrrolidinyl, piperidinyl, morpholinyl or N'-(C₁-C₄-alkyl)piperazinyl groups, and X is a chloride, bromide or iodide ion.

8. A process as claimed in claims 1 to 7, wherein the phosphazenium and/or phosphazanium halides of the general formulae III and IV are employed in a ratio by weight with respect to the Lewis acid of phosphazenium halide III and/or phosphazanium halide IV : Lewis acid of from 1:0.01 to 1:10.

9. A process as claimed in claims 1 to 8, wherein at least one of the components of the phosphazenium halide III and/or phosphazanium halide IV/Lewis acid catalyst system used is an iodide.

10. A process as claimed in claims 1 to 9, wherein a solubilizer is present.

11. A process as claimed in claims 1 to 9, wherein a solubilizer is absent.

12. A process as claimed in claims 1 to 11, wherein a zinc halide is used as Lewis acid.

13. A process as claimed in claims 1 to 11, wherein an organometallic Lewis acid is used.

14. A continuous embodiment of the process as claimed in claim 1, wherein an epoxybutene of the general formula II is added to a mixture of a phosphazenium halide or phosphazanium halide or of phosphazenium halide/phosphazanium halide mixtures with a Lewis acid in the presence or absence of an organic solubilizer at the reaction temperature, and the dihydrofuran I formed in the reaction is continuously distilled out during the reaction.

15. A process as claimed in claims 1 to 14, wherein vinyloxirane of the formula IIa 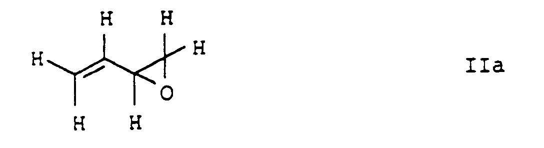 is employed as epoxybutene II.

## Revendications

1. Procédé de préparation de 2,5-dihydrofurannes de formule générale I 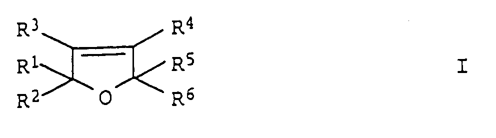 dans laquelle les restes R¹, R², R³, R⁴, R⁵ et R⁶ sont identiques ou différents et sont mis pour des atomes d'hydrogène ou des groupements alkyle en C₁-C₄, par transposition catalytique de 3,4-époxy-1-butènes de formule générale II 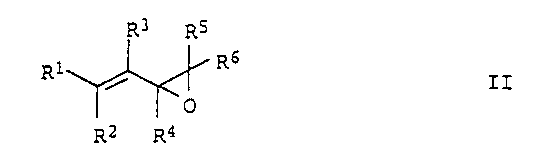 en présence d'un acide de Lewis ou d'iode à l'état d'élément ainsi qu'en présence ou en absence d'un agent solubilisant à une température de 60 à 200°C, caractérisé en ce que la réaction est effectuée en présence d'un halogénure de phosphazénium ou d'un halogénure de phosphazanium ou en présence de mélanges d'halogénures de phosphazénium et de phosphazanium.

2. Procédé selon la revendication 1, caractérisé en ce que l'on effectue la réaction en présence d'un halogénure de phosphazénium de formule générale III 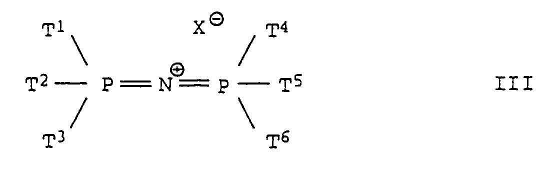 dans laquelle X représente un on chlorure, bromure ou iodure et dans laquelle les substituants T¹, T², T³, T⁴, T⁵ et T⁶ sont identiques ou différents et sont mis indépendamment les uns des autres pour des groupements alkyle en C₁-C₂₀ éventuellement substitués, des groupements aryle en C₆-C₁₀ éventuellement substitués, des groupements aralkyle en C₇-C₁₂ éventuellement substitués, des groupements di(alkyle en C₁-C₁₀)amino, des groupements di(cycloalkyle en C₃-C₈)amino, des groupements di(aryle en C₆-C₁₀)amino, des groupements (alkyle en C₁-C₁₀)(aryle en C₆-C₁₀)amino, des groupements di(aralkyle en C₇-C₁₂)amino, des groupements (alkyle en C₁-C₁₀)(aralkyle en C₇-C₁₂)amino, des groupements (aryle en C₆-C₁₀)-(aralkyle en C₇-C₁₂)amino, des groupements pyrrolidinyle, pipéridinyle, morpholinyle ou N'-(alkyle en C₁-C₄)pipérazinyle.

3. Procédé selon la revendication 1 et 2, caractérisé en ce que l'on effectue la réaction en présence d'un halogénure de phosphazénium de formule générale IIIa 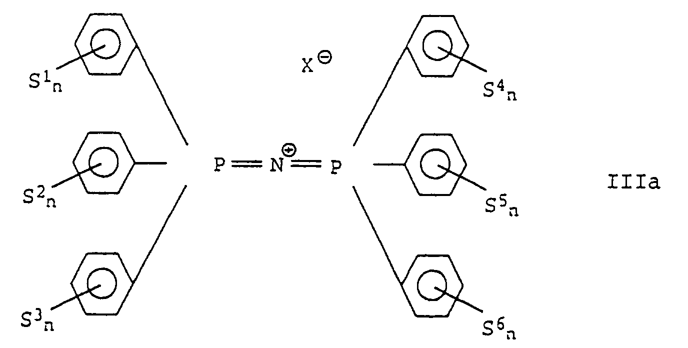 dans laquelle X est mis pour un ion chlorure, bromure ou iodure, chacun des 6 noyaux des groupements phényle de l'halogénure de phosphazénium IIIa, pris isolément, étant soit non substitué, soit portant 1 à 3 des substituants S¹, S², S³, S⁴, S⁵ ou S⁶, identiques ou différents, où S¹, S², S³, S⁴, S⁵ ou S⁶ représentent des groupements alkyle en C₁-C₂₀, des groupements alcoxy en C₁-C₂₀, des atomes de chlore et/ou de brome et n représente le nombre de substituants S¹, S², S³, S⁴, S⁵ ou S⁶ sur chacun des noyaux des groupements phényle et prend une valeur entière de 0 à 3.

4. Procédé selon l'une quelconque des revendications 1 à 3, caractérisé en ce que l'on effectue la réaction en présence d'un halogénure de phosphazénium de formule IIIb 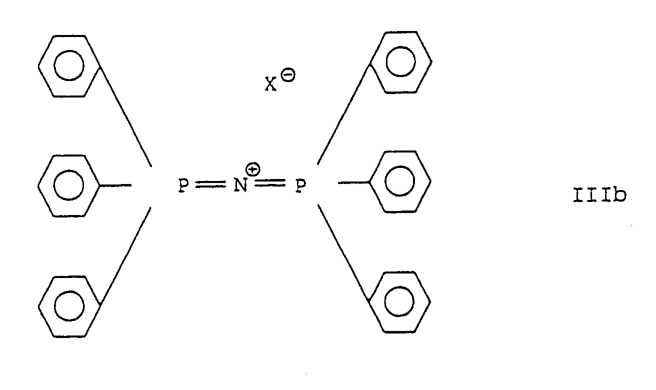 dans laquelle X est mis pour un on chlorure, bromure ou iodure.

5. Procédé selon la revendication 1 et 2, caractérisé en ce que l'on effectue la réaction en présence d'un halogénure de phosphazénium de formule IIIc 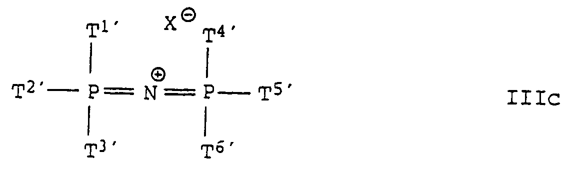 dans laquelle les restes T¹', T²', T³', T⁴', T⁵' et T⁶' sont identiques ou différents et sont mis, indépendamment les uns des autres, pour des groupements di(alkyle en C₁-C₄)amino ou pour des groupements pyrrolidinyle, pipéridinyle, morpholinyle ou N'-(alkyle en C₁-C₄)pipérazinyle et X est un ion chlorure, bromure ou iodure.

6. Procédé selon la revendication 1, caractérisé en ce que l'on effectue la réaction en présence d'un halogénure de phosphazanium de formule IV 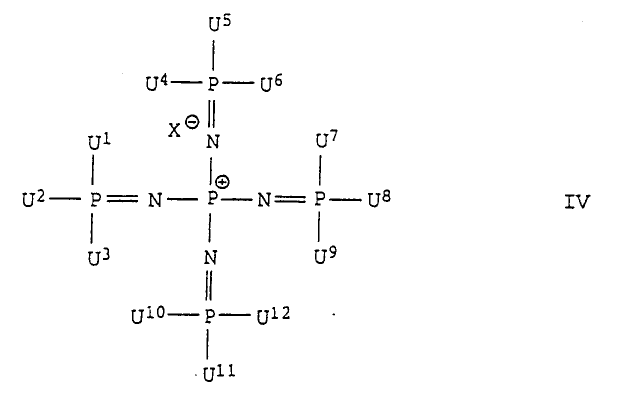 dans laquelle les substituants U¹, U², U³, U⁴, U⁵, U⁶, U⁷, U⁸, U⁹, U¹⁰, U¹¹ et U¹² sont identiques ou différents et, indépendamment les uns des autres, sont mis pour des groupements alkyle en C₁-C₂₀ éventuellement substitués, des groupements aryle en C₆-C₁₀ éventuellement substitués, des groupements aralkyle en C₇-C₁₂ éventuellement substitués, des groupements di(alkyle en C₁-C₁₀)amino, des groupements di(cycloalkyle en C₃-C₈)amino, des groupements di(aryle en C₆-C₁₀)amino, des groupements (alkyle en C₁-C₁₀)(aryle en C₆-C₁₀)amino, des groupements di(aralkyle en C₇-C₁₂)amino, des groupements (alkyle en C₁-C₁₀)(aralkyle en C₇-C₁₂)amino, des groupements (aryle en C₆-C₁₀)(aralkyle en C₇-C₁₂)amino, des groupements pyrrolidinyle, pipéridinyle, morpholinyle ou N'-(alkyle en C₁-C₄)pipérazinyle.

7. Procédé selon l'une quelconque des revendications 1 à 6, caractérisé en ce que l'on effectue la réaction en présence d'un halogénure de phosphazanium de formule IV, les substituants U¹, U², U³, U⁴, U⁵, U⁶, U⁷, U⁸, U⁹, U¹⁰, U¹¹ et U¹², étant identiques ou différents et étant mis, indépendamment les uns des autres, pour des groupements di(alkyle en C₁-C₄)amino, pyrrolidinyle, pipéridinyle, morpholinyle ou N'-(alkyle en C₁-C₄)pipérazinyle et X est un ion chlorure, bromure ou iodure.

8. Procédé selon l'une quelconque des revendications 1 à 7, caractérisé en ce que l'on utilise les halogénures de phosphazénium et/ou de phosphazanium de formules générales III et IV et l'acide de Lewis dans un rapport en poids halogénure de phosphazénium III et/ou halogénure de phosphazanium IV : acide de Lewis allant de 1 :0,01 à 1 : 10.

9. Procédé selon l'une quelconque des revendications 1 à 8, caractérisé en ce qu'au moins un des composants du système catalytique utilisé halogénure de phosphazénium III et/ou halogénure de phosphazanium IV/acide de Lewis est un on iodure.

10. Procédé selon l'une quelconque des revendications 1 à 9, caractérisé en ce que l'on travaille en présence d'un agent solubilisant.

11. Procédé selon l'une quelconque des revendications 1 à 9, caractérisé en ce que l'on travaille en l'absence d'agent solubilisant.

12. Procédé selon l'une quelconque des revendications 1 à 11, caractérisé en ce que l'on utilise un halogénure de zinc en tant qu'acide de Lewis.

13. Procédé selon l'une quelconque des revendications 1 à 11, caractérisé en ce que l'on utilise un acide de Lewis organométallique.

14. Réalisation en continu du procédé selon la revendication 1, caractérisé en ce que l'on met en réaction un époxybutène de formule générale II avec un mélange constitué d'un halogénure de phosphazénium ou d'un halogénure de phosphazanium ou constitué de mélanges halogénure de phosphazénium/halogénure de phosphazanium avec un acide de Lewis en présence ou en l'absence d'un agent solubilisant organique, à la température réactionnelle, et en ce que l'on élimine par distillation continue pendant la réaction, le dihydrofuranne I formé lors de la réaction.

15. Procédé selon l'une quelconque des revendications 1 à 14, caractérisé en ce que l'on utilise, en tant qu'époxybutène II, le vinyloxiranne de formule IIa.
